# EUROPEAN PATENT SPECIFICATION

(11) **EP 3 386 323 B1**
(45) Date of publication and mention of the grant of the patent: **03.02.2021**
(21) Application number: 16809444.9
(22) Date of filing: 15.12.2016
(51) Int. Cl.: A24F 47/00, A61M 11/04, A61M 15/00, A61M 15/06, A61M 16/00

(54) **PERSONAL VAPORIZER DEVICE**
PERSÖNLICHE VERDAMPFERVORRICHTUNG
DISPOSITIF DE VAPORISATION PERSONNEL

(30) Priority: 18.12.2015 EP 15201281
(43) Date of publication of application: 17.10.2018
(60) Divisional application: 20214564.5
(73) Proprietor: JT International S.A., 1202 Geneva (CH)
(72) Inventor: JAMES, Aled, Dorridge B93 8RR (GB); THOMAS, Richard, Leamington Spa CV32 4SA (GB); MAY, James, Kenilworth CV8 1BS (GB)
(74) Representative: Hoffmann Eitle
(86) International application number: PCT/EP2016/081231
(87) International publication number: WO 2017/102969

(56) References cited:
- EP-A1- 3 009 017
- CN-U- 204 157 645
- US-A1- 2005 016 533
- US-A1- 2011 036 346
- US-A1- 2014 366 898

## Description

### Field of the Invention

The present invention relates to a personal vaporizer device, such as an electronic smoking article.

### Background of the Invention

Personal vaporizer devices, such as electronic cigarettes or "e-cigarettes" as they are also known, have gained in popularity over the past ten years as an alternative to traditional smoking articles, like cigarettes, cigars, and cigarillos. Because the technology employed in personal vaporizer devices is still quite young, however, developments in the design and configuration of such devices are on-going to improve their performance and their reliability, as well as their ease of use, ease of production, and their production costs.

In this context, CN 204 157 645 U discloses a kind of electronic cigarette, comprising an electronic cigarette body, wherein the electronic cigarette body is provided with a smoke absorbing end, a tobacco tar bottle for storing tobacco tar, an atomization assembly for atomizing cigarette oil, which is electrically connected with an atomizing component and a battery for powering an atomizing component and a display module for showing electronic cigarette information.

Further, EP 3 009 017 A shows a capsule containing a liquid to be supplied to an atomizer and being mounted on or in an electronic smoking device. The capsule has a controller including a memory, which receives data from and transmits data to control electronics of the electronic smoking device. A capacitor in the capsule is charged by the electronic smoking device and powers the controller during intermediate intervals. Electrical contacts of the capsule are connected to electrical contacts of the electronic smoking device to charge the capacitor and exchange data.

### Summary of the Invention

In view of the above, an object of the invention is to provide a new and improved personal vaporizer device, especially an improved electronic smoking article. In particular, it would be desirable to provide such a personal vaporizer device which is more ergonomic and user-friendly for a user. It would also be useful to provide such a personal vaporizer device which protects sensitive or replaceable parts or components of the device, such as a cartridge or capsule for holding a liquid to be vaporized, especially a disposable cartridge or a refillable cartridge.

In accordance with the present invention, a personal vaporizer device, especially an electronic smoking article, as recited in claim 1, as well as a method of installing a cartridge in a personal vaporizer device as recited in claim 14, are provided. Various preferred and/or advantageous features of the invention are recited in the dependent claims. According to an aspect, the invention provides a personal vaporizer device, especially an electronic smoking article, comprising: a removable cartridge which includes a reservoir for storing a liquid to be vaporized; and an elongate body portion to which the removable cartridge is connected. The elongate body portion includes a cover member which is movable in a longitudinal direction of the body portion between a first position and a second position, wherein the removable cartridge is at least partially, and optionally substantially entirely, covered or obscured by the cover member in the first position. As noted above, the cover member may thus provide a physical barrier for protecting the removable cartridge in the first position.

In relation to the cartridge being at least partially covered or obscured by the cover member in the first position, it will be noted that the cover member typically covers or obscures at least 50% of an outer surface of the cartridge in the first position, and more preferably at least 80% of an outer surface of the cartridge in the first position. In a preferred embodiment, the cover member covers or obscures in the range of 80% to 100% of the cartridge in the first position.

In a preferred embodiment, the cover member may substantially cover or obscure a mouthpiece of the personal vaporizer device in the first position. In this way, the first position may be clearly designed or intended to form a non-use position or a non-activated position for the vaporizer device. The mouthpiece may, for example, be provided on the cartridge. In this context, the first position of the cover member may be a substantially extended position, in which the cover member extends in the longitudinal direction of the body portion to cover the cartridge and/or mouthpiece of the personal vaporizer device.

In a preferred embodiment the cover member comprises at least one of a front cover panel, which extends over a front of the elongate body portion, and a rear cover panel, which extends over a rear of the elongate body portion. In this way, the cover member may form part of an outer casing of the elongate body portion.

In a preferred embodiment, a volume of liquid in the reservoir of the cartridge is or remains visible when the cover member is in the first position. That is, although the cartridge is largely covered or obscured by the cover member in this position, a user is nevertheless able to check and see how much liquid is in the reservoir of the cartridge. In this regard, a window could be provided in the cover member. As an alternative, or in addition, the cover member need not fully enclose or encase the cartridge, thereby leaving an area or region uncovered for a user to determine visually what volume of liquid remains in the reservoir. For example, a side region of the cartridge could optionally remain uncovered by the cover member.

In a preferred embodiment, the second position of the cover member may assume a substantially retracted position, with the cover member configured and arranged to leave the removable cartridge substantially uncovered or unobscured in the second position. That is, the cover member may be retracted from the removable cartridge in the longitudinal direction to the second position. Thus, this second position may be designed or intended to form a use position or an activated position for the vaporizer device. Further, the second position may be particularly suitable for connecting a removable cartridge to and/or for removing a removable cartridge from the body portion of the personal vaporizer device.

In a preferred embodiment, the personal vaporizer device comprises guide means for guiding movement of the cover member in the longitudinal direction between the first position and the second position. In this regard, the cover member may, for example, be mounted on one or more tracks or rails provided in or on the body portion for movement of the cover member in the longitudinal direction between the first position and the second position. That is, the one or more tracks or rails may define a path of travel for the cover member between the first position and the second position. The cover member therefore preferably includes one or more complementary follower element configured for engaging a respective track or rail for following same along the predefined path of travel between the first and second positions. The movement of the cover member in the longitudinal direction may be a sliding movement, although rolling movement or other translational movement is also contemplated.

In a preferred embodiment, the elongate body portion includes a switch which is configured to activate one or more functionality of the personal vaporizer device. In this regard, the switch is preferably provided either on the movable cover member or in operative connection with the cover member. In this way, the one or more functionality of the device may, for example, be activated via the switch when the cover member is moved to the first position and/or to the second position. For example, the device may include two separate functions which could be activated by movement of the cover member, with one function being activated when the cover member is in the first position, and another function being activated when the cover member is in the second position. Alternatively, or in addition, multiple functions could be activated in each of the first and/or second positions.

In a preferred embodiment, the elongate body portion comprises a power supply unit, especially a battery unit, for supplying electrical power to the device. In this regard, the personal vaporizer device will typically include a vaporizer unit for vaporizing the liquid from the reservoir to be inhaled by a user. Furthermore, the vaporizer device may optionally include a control unit for controlling operation of the device. Thus, the power supply unit is desirably designed to supply electrical power to the vaporizer unit and/or to the control unit. In a particularly preferred embodiment, the vaporizer unit and/or the control unit may be incorporated in the cartridge which is configured to be connected to the elongate body portion of the device. Accordingly, in a preferred embodiment the vaporizer unit comprises a heater for heating the liquid to be vaporized to generate the vapour to be inhaled, and a liquid delivery means which is configured to convey the liquid from the reservoir to the heater for vaporization.

As noted above, the cartridge is preferably configured to be connected to an end region of the elongate body portion in the vaporizer device. The end region of the elongate body portion thus preferably includes one or more electrical connectors for making an electrical connection with one or more complementary electrical connectors provided on the cartridge.

Furthermore, in a preferred embodiment, the body portion has one or more indicator, especially an illuminated indicator, such as an LED, for indicating an operational status of the device and/or for indicating available power supply in a power supply unit of the elongate body portion.

According to a further aspect, the present invention provides a personal vaporizer device, especially an electronic smoking article, configured to receive a removable cartridge with a reservoir for holding a liquid to be vaporized. The vaporizer device comprises an elongate body portion to which the cartridge is configured to be connected. The elongate body portion includes a power supply unit, especially a battery unit, for supplying electrical power to the device and at least one part of a controller or control unit for controlling operation of the device. A user interface for the control unit or controller is provided on an outer cover member of the elongate body portion and includes one or more user-interface elements over a longitudinal extent of the elongate body portion.

In a preferred embodiment, the control unit or controller is configured to manage one or more of: a heater or heating function of the device; a power supply function of the device; and a capsule recognition function of the device. The control unit or controller typically comprises a user interface for the device. Preferably, one or more LEDs are provided to act or to serve as illuminated indicator elements for indicating an operational status of the device and/or for indicating available power supply in the power supply unit.

In a preferred embodiment, the personal vaporizer device comprises one or more sensors for determining one or more operating condition of the device. The sensor or sensors may be incorporated in the cartridge and/or in the body portion of the device. The sensor(s) may, for example, include a temperature sensor, a liquid volume sensor, a puff sensor, and/or gyroscopic sensors.

According to a further aspect, the invention also provides a method of installing a cartridge in a personal vaporizer device, comprising the steps of:
providing a personal vaporizer device having an elongate body portion to which a removable cartridge is configured to be connected, the removable cartridge including a reservoir for storing a liquid to be vaporized and a mouthpiece provided on the removable cartridge;
providing a cover member on the elongate body portion which is movable in a longitudinal direction of the body portion between a first extended position and a second retracted position;
moving the cover member to the second retracted position to access an end region of the elongate body portion; and
attaching the removable cartridge to the accessed end region of the elongate body portion of the personal vaporizer device.

In an embodiment of the method, after attaching the removable cartridge to the end region of the elongate body portion, the cover member is moved to the first position to substantially cover the removable cartridge. As noted above, this has the effect of protecting the installed removable cartridge from external influences.

In an embodiment of the method, after attaching the removable cartridge to the end region of the elongate body portion, movement of the cover member between the first position and the second positon may act to switch the personal vaporizer device between an activated and a deactivated state.

### Brief Description of the Drawings

For a more complete understanding of the invention and the advantages thereof, exemplary embodiments of the invention are explained in more detail in the following description with reference to the accompanying drawing figures, in which like reference characters designate like parts and in which:
- Fig. 1: is a side view of a personal vaporizer device, especially an electronic smoking article, according to an embodiment, with a cover member of the device shown in a first position;
- Fig. 2: is a side view of the personal vaporizer device shown in Fig. 1, with the cover member of the device shown in a first position;
- Fig. 3: is a perspective view of a cartridge for a personal vaporizer device according to an embodiment;
- Fig. 4: is a schematic sectioned side view of a cartridge with vaporizer unit installed in a personal vaporizer device via a first type of connection;
- Fig. 5: is a schematic sectioned side view of a cartridge with vaporizer unit installed in a personal vaporizer device via a second type of connection;
- Fig. 6: is a schematic sectioned side view of a cartridge with vaporizer unit installed in a personal vaporizer device via a third type of connection;
- Fig. 7: is a perspective view of the personal vaporizer device illustrated in Figs. 1 and 2, with the cover member shown in the first position;
- Fig. 8: is a perspective view of the cover member of the personal vaporizer device shown in Figs. 1, 2 and 7;
- Fig. 9: is a perspective view of an underside of the cover member shown in Fig. 8;
- Fig. 10: is a perspective view of the personal vaporizer device shown in Fig. 7 with the cover member represented transparent providing a view of the control unit and user interface elements;
- Fig. 11: is a flow diagram which schematically represents a method according to an embodiment of the invention.

The accompanying drawings are included to provide a further understanding of the present invention and are incorporated in and constitute a part of this specification. The drawings illustrate particular embodiments of the invention and together with the description serve to explain the principles of the invention. Other embodiments of the invention and many of the attendant advantages of the invention will be readily appreciated as they become better understood with reference to the following detailed description.

It will be appreciated that common and/or well understood elements that may be useful or necessary in a commercially feasible embodiment are not necessarily depicted in order to facilitate a more abstracted view of the embodiments. The elements of the drawings are not necessarily illustrated to scale relative to each other. It will further be appreciated that certain actions and/or steps in an embodiment of a method may be described or depicted in a particular order of occurrences while those skilled in the art will understand that such specificity with respect to sequence is not actually required. It will also be understood that the terms and expressions used in the present specification have the ordinary meaning as is accorded to such terms and expressions with respect to their corresponding respective areas of inquiry and study, except where specific meanings have otherwise been set forth herein.

### Detailed Description of Embodiments

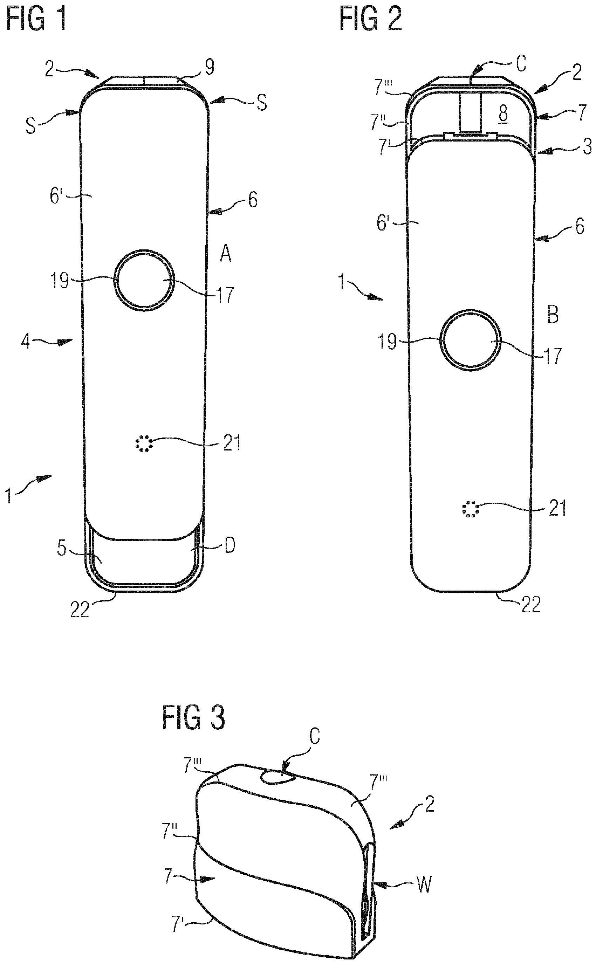

With reference firstly to Figs. 1 and 2 of the drawings, a personal vaporizer device 1 in the form of electronic cigarette or "e-cigarette" is illustrated in different positions and in partial cross-section. The device 1 includes a replaceable cartridge 2 which is attached at one end region 3 of an elongate body 4. The elongate body 4 in turn comprises a casing 5 which encloses a power supply unit in the form of a battery unit D for supplying electrical power to the device 1. The casing 5 of the elongate body 4 includes a cover member 6 which is movable in a longitudinal direction of the body 4 between a first position A (i.e. shown in Fig. 1) and a second position B (i.e. shown in Fig. 2). In this regard, the cover member 6 comprises a front cover panel 6' which extends over a front side of the body 4, and a rear cover panel 6", which extends over a rear side of the body 4.

As can be seen in drawing Figs. 2, the cartridge 2 comprises a housing 7 that encloses a reservoir 8 for storing a liquid to be vaporized. The housing 7 has a flat base 7' and generally vertically extending sides 7" which transition at curved upper shoulder regions 7'" to form or terminate at a mouthpiece 9 at a top of the cartridge 2. In this way, the shell of the housing 7 encloses the reservoir 8 to form a small tank for storing the liquid (i.e. of a type known in the art) to be vaporized for inhalation by a user of the e-cigarette. Furthermore, the cartridge 2 includes an airflow channel C which extends centrally through the housing 7 from the base 7' to the top and is configured to guide the vapour to the mouthpiece 9 for inhalation by the user. The airflow channel C has a generally circular cross-section and is surrounded by the reservoir 8. In order to generate the vapour, the cartridge 2 incorporates a vaporizer unit having an electric heater with a heating element or wire provided wound in a coil within the airflow channel C for heating the liquid to be vaporized to generate the vapour to be inhaled. The vaporizer unit (not shown) further comprises a liquid delivery means which is configured to convey the liquid from the reservoir 8 to the heating wire for vaporization.

Referring to drawing Figs. 1 and 2, the cover member 6, which is comprised of the front and rear panel members 6', 6", is movable in a longitudinal direction of the device body 4 - e.g. by sliding or in translation - between the first position A shown in Fig. 1 and the second position B shown in Fig. 2. In the first extended position A, the replaceable cartridge 2 comprising the reservoir 8 and the vaporizer unit 10 is substantially covered or obscured by the cover member 6. In this way, the cover member 6 provides a physical barrier for protecting the cartridge 2 in the first position A. Nevertheless, the volume or amount of liquid in the reservoir 8 of the cartridge 2 remains visible when the cover member 6 is in the first position A. That is, although the cartridge 2 is largely covered or obscured by cover member 6 in this position, a window may be provided in the cover member 6 and/or a side region S of the cartridge 2 remains uncovered by the cover member 6 and may include a window W for a user to determine visually how much liquid remains in the reservoir 8.

For sliding or moving the cover member 6 relative to the remainder of the casing 5 between the first and second positions A, B, at least one or both of the front and rear panel members 6', 6" may be held and engaged between a thumb and finger of the user. In the second position B, the cover member 6 is retracted to leave the cartridge 2 either partially or substantially uncovered or unobscured. That is, in the second position B the cover member 6 is retracted from the cartridge 2 in the longitudinal direction to a use position or an activated position for the e-cigarette or vaporizer device 1. To this end, the body 4 includes a switch 17 provided in the form of a round button set within an opening 18 in the cover member 6. The switch or button 17 is configured to activate the device 1 and the switch 17 is in operative connection with a part of the controller or control unit below the cover member 6. In this regard, the device 1 may be designed to only be able to be activated via the switch 17 when the front and rear panels 6', 6" are moved from the first position A to the second position B. The switch 17 is surrounded by a ring-shaped indicator 19 which illuminates on the front panel 6' for indicating an operational status of the device 1. These elements of the vaporizer device 1 will be described in greater detail below.

In addition, the second position B of the cover member 6 is designed for removing the cartridge 2 from, and/or for connecting a cartridge 2 to, the body portion 4 of the personal vaporizer device or e-cigarette 1. As noted above, the cartridge 2 is configured to be connected to the end region 3 of the body portion 4. This end region 3 of the elongate body 4 thus preferably includes one or more electrical contacts 14 for making an electrical connection with one or more complementary electrical connectors provided on the cartridge 2 for connecting electrical power to the heater of the vaporizer unit within the cartridge 2.

Drawing Fig. 3 shows a perspective view of a cartridge 2 according to a similar embodiment to the one described above. As described previously, the cartridge 2 here typically includes a housing 7 enclosing a reservoir 8 for storing the liquid to be vaporized and defining an airflow channel C which extends centrally through the housing 7 to a mouthpiece 9 at a top of the cartridge 2. Further, the cartridge 2 includes a vaporizer unit (not shown) having an electric heater with a heating element provided wound in a coil within the airflow channel C for heating the liquid to be vaporized to generate the vapour to be inhaled. The vaporizer unit comprises a liquid delivery means configured to convey the liquid from the reservoir 8 via capillary action or other known mechanisms.

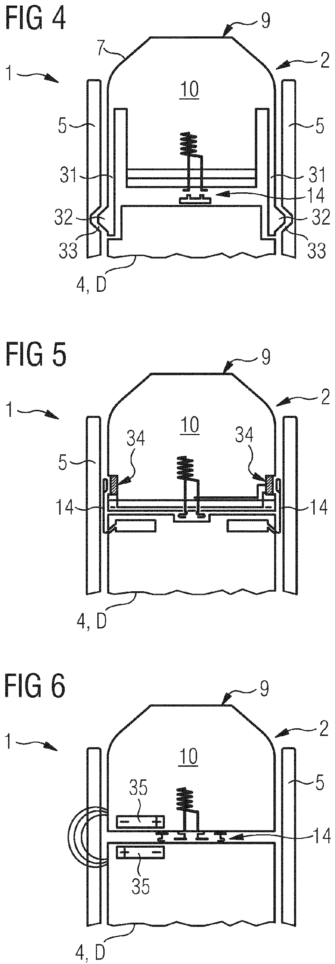

Referring now to Figs. 4 to 6 of the drawings, three different types of mechanical and electrical connections between the cartridge 2 and power supply (e.g. battery unit D) via the end region 3 of the body 4 of the personal vaporizer device 1 are schematically illustrated. In each case, the cartridge 2 is configured for a push-on/pull-off type connection into the open upper end region 3 of the casing 5 of the device. In Fig. 4, the sides 7" of the cartridge housing 7 include resilient or flexible members 31 (e.g. formed as leaf-springs or cantilevers) having projecting lugs 32 for receipt or engagement in complementary recesses 33 formed on an inner side of the casing 5. Electrical contact elements 14 are provided separately for power supply to the controller 13 and heater via electrical contacts provided below the cartridge 2. In the embodiment of Fig. 5, the sides 7" of the cartridge housing 7 are received in the open upper end region 3 of the casing 5 and resilient electrical contact elements 14 extend upwards from the power supply and engage electrical contacts 34 provided in the sides 7" of the cartridge housing 7. In the embodiment of Fig. 6, by contrast, a mechanical connection is effected via magnetic connector elements 35 provided on both the cartridge 2 and the body part 4 of the personal vaporizer device 1 at the end region 3. The electrical connection again occurs separately via electrical contacts 14 below the base 7' of the cartridge housing 7.

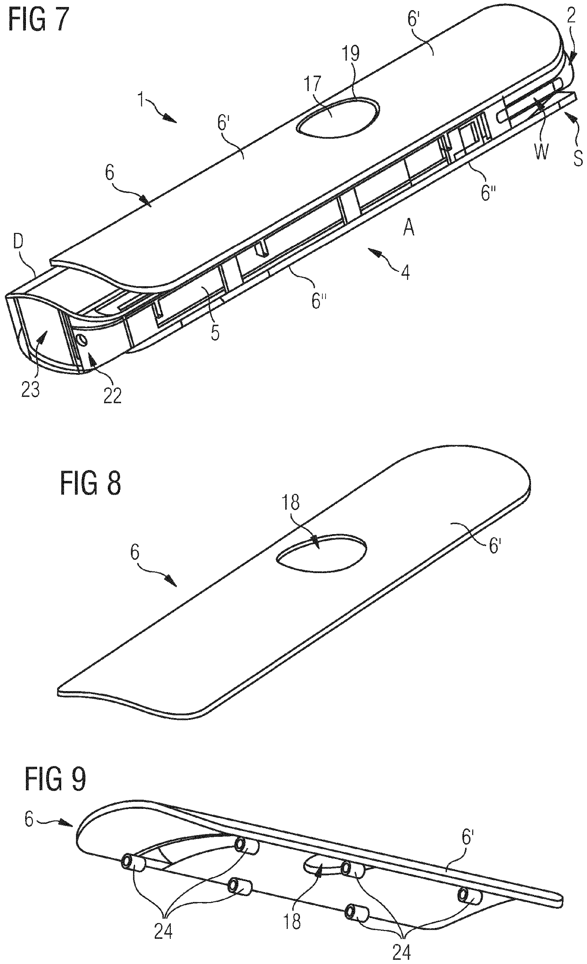

With reference now to Figs. 7 to 9 of the drawings, the geometry and construction of the cover member 6 can be more clearly seen. In particular, the front panel 6' of the cover member is functionalized by its convex outer shape, its thickness and flexibility. In particular, the panel 6' is not only configured to move between the first and second positions A, B but may also bend and form as a user interface for operation of the device 1. In this regard, it has already been noted above that the front panel 6' of the cover member 6 includes user interface elements, such as the switch 17 in the form of a button set within the opening 18 in the cover member 6 and surrounded by a ring-shaped indicator 19 which illuminates the front panel 6' for indicating an operational status of the device 1. The switch or button 17 which is configured to activate the device 1 for vapour generation after the cover member 6 has been moved to the second or "in-use" position B. The front panel 6' may also include one or more further switch or button 21 which may be positioned on a surface thereof (e.g. discretely or clearly) for activating additional functionalities, such as for setting an operating mode. Each of the switches or buttons 17, 21 is in operative connection with a part of the controller or control unit 13 of the device 1 below the cover member 6, which is described later. When the cover member 6 is moved to the second position B, the indicator 17 may illuminate to identify that the device 1 has been activated and/or is ready for operation. Further, the indicator 20 may illuminate in a different colour and/or in a different manner (e.g. blink or pulsate) to signal to a user that the available power supply in the battery unit D is low (e.g. that the battery unit D should be recharged). For this purpose, the opposite end region 22 of the body 4 of the e-cigarette 1 typically includes a connector 23 for connection to a re-charging dock or re-charging station and/or for connection of a re-charging cable.

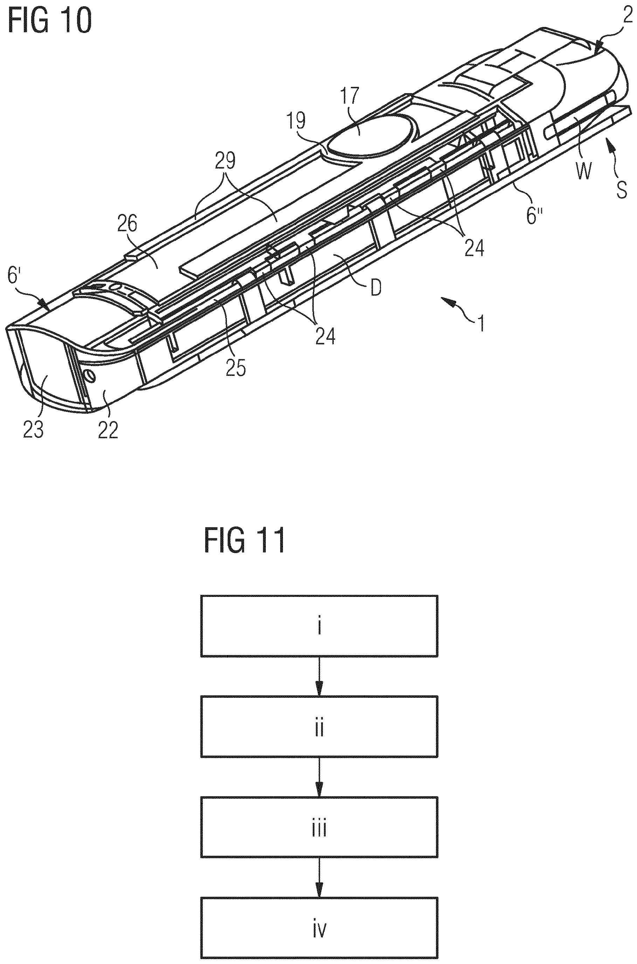

As is apparent from drawing Fig. 9, the underside of the front panel 6' includes sleeve elements 24 for sliding engagement with parallel rail members 25 (seen in Fig. 10) on the body portion 4 during longitudinal movement between the first and second positions A, B. In this way, the rail members 25 function as guide means for guiding the longitudinal movement of the cover member 6 and sleeve elements 24 form follower elements for following the path defined by the rails. Further, referring to drawing Fig. 10, the elongate body portion 4 of the device 1 includes a circuit board (not shown) mounted below the front panel 6' which forms part of the controller or control unit 13 of the e-cigarette.

Finally, referring to Fig. 11 of the drawings, a flow diagram is shown that illustrates schematically the steps in a method of installing a cartridge in a personal vaporizer device 1, especially in an electronic smoking article according to the embodiments of the invention described above with respect to Figs. 1 to 10. In this regard, the first box i of Fig. 11 thus represents the step of providing a personal vaporizer device 1 having an elongate body portion 4 to which a removable cartridge 2 is configured to be connected, the cartridge 2 including a reservoir 8 for storing a liquid to be vaporized. The second box ii represents the step of providing a cover member 6 on the elongate body portion 4 which is movable in a longitudinal direction of the body portion 4 between a first extended position A and a second retracted position B. The third box iii represents the step of moving cover member 6 to the second retracted position B to access an end region 3 of the body portion 4. The final box iv in Fig. 11 of the drawings represents the step of attaching the cartridge 2 to the accessed end region 3 of the body portion 4 personal vaporizer device 1.

### List of Drawing Signs

- 1: personal vaporizer device or e-cigarette
- 2: cartridge
- 3: end region of elongate body
- 4: elongate body
- 5: casing
- 6: cover member
- 6': front panel
- 6": rear panel
- 7: housing
- 7': housing base
- 7": housing sides
- 7'": housing shoulder region
- 8: reservoir
- 9: mouthpiece
- 10: vaporizer unit
- 14: electrical contact
- 15: conductor element
- 17: switch or button
- 18: opening in the cover member
- 19: ring-shaped indicator
- 21: mode switch or button
- 22: opposite end region of body portion
- 23: charging connector
- 24: sleeve element
- 25: rail member
- 26: circuit board
- 29: light-guide element
- 31: resilient or flexible member
- 32: projecting lug
- 33: recess
- 34: electrical contact
- 35: magnetic connector element
- A: first position of cover member
- B: second position of cover member
- D: battery unit
- C: airflow channel
- S: sides of the casing
- W: window

## Claims

1. A personal vaporizer device (1), comprising:
a cartridge (2) which includes a reservoir (8) for storing a liquid to be vaporized, and
an elongate body portion (4) to which the cartridge (2) is connected, wherein the elongate body portion (4) comprises a cover member (6) which is movable in a longitudinal direction of the elongate body portion (4) between a first position (A) and a second position (B),
wherein the cartridge (2) is at least partially covered or obscured by the cover member (6) in the first position (A), **characterised in that**
the cartridge (2) comprises a housing (7) that encloses the reservoir (8), the cartridge (2) is configured to be connected to an end region (3) of the elongate body portion (4) and wherein a mouthpiece (9) is provided on the cartridge (2).

2. A personal vaporizer device (1) according to claim 1, wherein a volume of liquid in the reservoir (8) of the cartridge (2) remains visible when the cover member (6) is in the first position (A).

3. A personal vaporizer device (1) according to claim 2, wherein a window is provided in the cover member (6) and/or a side region (S) of the cartridge (2) remains uncovered by the cover member (6) for a user to determine visually how much liquid remains in the reservoir (8) of the cartridge (2) when the cover member (6) is in the first position (A).

4. A personal vaporizer device (1) according to any preceding claim, wherein the cartridge (2) includes a window (W) for a user to determine visually how much liquid remains in the reservoir (8) of the cartridge (2), the window (W) preferably being provided on a side region (S) of the cartridge (2).

5. A personal vaporizer device (1) according to any preceding claim, wherein the second position (B) of the cover member (6) is a substantially retracted position in which the cartridge (2) is substantially uncovered or unobscured by the cover member (6), for connection of the cartridge (2) to, and/or removal of the cartridge (2) from, the elongate body portion (4), the second position (B) preferably being a position in which the personal vaporizer device (1) is activated.

6. A personal vaporizer device (1) according to any preceding claim, wherein the first position (A) of the cover member (6) is a substantially extended position, the first position (A) preferably being a position in which the personal vaporizer device (1) is deactivated.

7. A personal vaporizer device (1) according to any preceding claim, wherein the cover member (6) forms a part of an outer casing (5) of the elongate body portion (4).

8. A personal vaporizer device (1) according to claim 7, wherein the cover member (6) comprises at least one of a front cover panel (6') which extends over a front of the elongate body portion (4) and a rear cover panel (6") which extends over a rear of the elongate body portion (4).

9. A personal vaporizer device (1) according to any preceding claim, further comprising at least one sensor for determining one or more operating conditions of the personal vaporizer device (1), wherein the at least one sensor is incorporated in the cartridge (2) and/or the elongate body portion (4), and wherein the at least one sensor comprises a temperature sensor, a liquid volume sensor, a puff sensor, or a gyroscopic sensor.

10. A personal vaporizer device (1) according to any preceding claim, wherein the cover member (6) covers or obscures at least 50% of an outer surface of the cartridge (2) in the first position (A), preferably at least 80% of an outer surface of the cartridge (2), more preferably in the range of 80% to 100% of the cartridge (2).

11. A personal vaporizer device (1) according to any preceding claim, wherein the elongate body portion (4) comprises a switch (17) operatively connected with the cover member (6) and configured to activate at least one functionality when the cover member (6) is moved to the first position (A) and/or to the second position (B), and wherein the elongate body portion (4) comprises at least one indicator (19), preferably an illuminated indicator, such as an LED, for indicating an operational status of the personal vaporizer device (1).

12. A personal vaporizer device (1) according to claim 11, wherein the switch (17) is configured to activate the personal vaporizer device (1) for vapour generation after the cover member (6) has been moved to the second position (B), and wherein the at least one indicator (19) is configured to indicate that the personal vaporizer device (1) has been activated.

13. A personal vaporizer device (1) according to any preceding claim, further comprising guide means (25) for guiding movement of the cover member (6) in the longitudinal direction between the first position (A) and the second position (B).

14. A method of installing a removable cartridge (2) in a personal vaporizer device (1), especially in an electronic smoking article, comprising the steps of:
providing a personal vaporizer device (1) having an elongate body portion (4) to which the removable cartridge (2) is configured to be connected, the removable cartridge (2) including a housing that encloses a reservoir (8) for storing a liquid to be vaporized and a mouthpiece (9) provided on the removable cartridge (2);
providing a cover member (6) on the elongate body portion (4) which is movable in a longitudinal direction of the body portion (4) between a first extended position (A) and a second retracted position (B);
moving cover member (6) to the second retracted position (B) to access an end region (3) of the elongate body portion (4); and
attaching the removable cartridge (2) to the accessed end region (3) of the elongate body portion (4) of the personal vaporizer device (1).

15. A method according to claim 14, comprising the step of: after attaching the removable cartridge (2) to the end region (3) of the elongate body portion (4), moving the cover member (6) to the first extended position (A) to substantially cover the removable cartridge (2).

## Patentansprüche

1. Persönliche Verdampfervorrichtung (1), umfassend:
eine Kartusche (2), die ein Reservoir (8) zum Aufbewahren einer zu verdampfenden Flüssigkeit umfasst, und
einen länglichen Körperabschnitt (4), mit dem die Kartusche (2) verbunden ist, wobei der längliche Körperabschnitt (4) ein Abdeckelement (6) umfasst, das in einer Längsrichtung des länglichen Körperabschnitts (4) zwischen einer ersten Position (A) und einer zweiten Position (B) bewegbar ist,
wobei die Kartusche (2) durch das Abdeckelement (6) in der ersten Position (A) mindestens teilweise abgedeckt oder verdeckt ist,
**dadurch gekennzeichnet, dass** die Kartusche (2) ein Gehäuse (7) umfasst, welches das Reservoir (8) umschließt, die Kartusche (2) konfiguriert ist, um mit einer Endregion (3) des länglichen Körperabschnitts (4) verbunden zu sein, und wobei ein Mundstück (9) an der Kartusche (2) bereitgestellt ist.

2. Persönliche Verdampfervorrichtung (1) nach Anspruch 1, wobei ein Flüssigkeitsvolumen in dem Reservoir (8) der Kartusche (2) sichtbar bleibt, wenn sich das Abdeckelement (6) in der ersten Position (A) befindet.

3. Persönliche Verdampfervorrichtung (1) nach Anspruch 2, wobei ein Fenster in dem Abdeckelement (6) bereitgestellt ist und/oder eine Seitenregion (S) der Kartusche (2) von dem Abdeckelement (6) unbedeckt bleibt, damit ein Benutzer visuell bestimmen kann, wie viel Flüssigkeit in dem Reservoir (8) der Kartusche (2) verbleibt, wenn sich das Abdeckelement (6) in der ersten Position (A) befindet.

4. Persönliche Verdampfervorrichtung (1) nach einem der vorstehenden Ansprüche, wobei die Kartusche (2) ein Fenster (W) umfasst, damit ein Benutzer visuell bestimmen kann, wie viel Flüssigkeit in dem Reservoir (8) der Kartusche (2) verbleibt, wobei das Fenster (W) bevorzugt auf einer Seitenregion (S) der Kartusche (2) bereitgestellt ist.

5. Persönliche Verdampfervorrichtung (1) nach einem der vorstehenden Ansprüche, wobei die zweite Position (B) des Abdeckelements (6) eine im Wesentlichen eingezogene Position ist, in der die Kartusche (2) zur Verbindung der Kartusche (2) mit dem länglichen Körperabschnitt (4) und/oder zur Entnahme der Kartusche (2) aus demselben von dem Abdeckelement (6) im Wesentlichen unbedeckt oder unverdeckt ist, wobei die zweite Position (B) bevorzugt eine Position ist, in der die persönliche Verdampfervorrichtung (1) aktiviert ist.

6. Persönliche Verdampfervorrichtung (1) nach einem der vorstehenden Ansprüche, wobei die erste Position (A) des Abdeckelements (6) eine im Wesentlichen ausgezogene Position ist, wobei die erste Position (A) bevorzugt eine Position ist, in der die persönliche Verdampfervorrichtung (1) deaktiviert ist.

7. Persönliche Verdampfervorrichtung (1) nach einem der vorstehenden Ansprüche, wobei das Abdeckelement (6) Teil einer Außenhülle (5) des länglichen Körperabschnitts (4) ist.

8. Persönliche Verdampfervorrichtung (1) nach Anspruch 7, wobei das Abdeckelement (6) mindestens eine von einer vorderen Abdeckplatte (6'), die sich über eine Vorderseite des länglichen Körperabschnitts (4) erstreckt, und einer hinteren Abdeckplatte (6"), die sich über eine Rückseite des länglichen Körperabschnitts (4) erstreckt, umfasst.

9. Persönliche Verdampfervorrichtung (1) nach einem der vorstehenden Ansprüche, ferner umfassend mindestens einen Sensor zum Bestimmen einer oder mehrerer Betriebsbedingungen der persönlichen Verdampfervorrichtung (1), wobei der mindestens eine Sensor in die Kartusche (2) und/oder den länglichen Körperabschnitt (4) integriert ist, und wobei der mindestens eine Sensor einen Temperatursensor, einen Flüssigkeitsvolumensensor, einen Zugsensor oder einen Gyroskopsensor umfasst.

10. Persönliche Verdampfervorrichtung (1) nach einem der vorstehenden Ansprüche, wobei das Abdeckelement (6) mindestens 50 % einer Außenfläche der Kartusche (2) in der ersten Position (A), bevorzugt mindestens 80 % einer Außenfläche der Kartusche (2), weiter bevorzugt in dem Bereich von 80 % bis 100 % der Kartusche (2), abdeckt oder verdeckt.

11. Persönliche Verdampfervorrichtung (1) nach einem der vorstehenden Ansprüche, wobei der längliche Körperabschnitt (4) einen Schalter (17) umfasst, der betriebsfähig mit dem Abdeckelement (6) verbunden ist und konfiguriert ist, um mindestens eine Funktionalität zu aktivieren, wenn das Abdeckelement (6) in die erste Position (A) und/oder die zweite Position (B) bewegt wird, und wobei der längliche Körperabschnitt (4) mindestens eine Anzeige (19), bevorzugt eine beleuchtete Anzeige, wie etwa eine LED, zum Angeben eines Betriebsstatus der persönlichen Verdampfervorrichtung (1) umfasst.

12. Persönliche Verdampfervorrichtung (1) nach Anspruch 11, wobei der Schalter (17) konfiguriert ist, um die persönliche Verdampfervorrichtung (1) zur Dampfgenerierung zu aktivieren, nachdem das Abdeckelement (6) in die zweite Position (B) bewegt wurde, und wobei die mindestens eine Anzeige (19) konfiguriert ist, um anzugeben, dass die persönliche Verdampfervorrichtung (1) aktiviert wurde.

13. Persönliche Verdampfervorrichtung (1) nach einem der vorstehenden Ansprüche, ferner umfassend Führungsmittel (25), um die Bewegung des Abdeckelements (6) in der Längsrichtung zwischen der ersten Position (A) und der zweiten Position (B) zu führen.

14. Verfahren zum Installieren einer abnehmbaren Kartusche (2) in einer persönlichen Verdampfervorrichtung (1), insbesondere bei einem elektronischen Rauchartikel, umfassend folgende Schritte:
Bereitstellen einer persönlichen Verdampfervorrichtung (1), die einen länglichen Körperabschnitt (4) aufweist, der zur Verbindung mit der abnehmbaren Kartusche (2) konfiguriert ist, wobei die abnehmbare Kartusche (2) ein Gehäuse, das ein Reservoir (8) zum Aufbewahren einer zu verdampfenden Flüssigkeit umschließt, und ein Mundstück (9), das an der abnehmbaren Kartusche (2) bereitgestellt ist, umfasst;
Bereitstellen eines Abdeckelements (6) an dem länglichen Körperabschnitt (4), der in einer Längsrichtung des Körperabschnitts (4) zwischen einer ersten ausgezogenen Position (A) und einer zweiten eingezogenen Position (B) bewegbar ist;
Bewegen des Abdeckelements (6) in die zweite eingezogene Position (B), um auf eine Endregion (3) des länglichen Körperabschnitts (4) zuzugreifen; und
Anbringen der abnehmbaren Kartusche (2) an der zugänglichen Endregion (3) des länglichen Körperabschnitts (4) der persönlichen Verdampfervorrichtung (1).

15. Verfahren nach Anspruch 14, umfassend folgenden Schritt: nach dem Anbringen der abnehmbaren Kartusche (2) an der Endregion (3) des länglichen Körperabschnitts (4), Bewegen des Abdeckelements (6) in die erste ausgezogene Position (A), um die abnehmbare Kartusche (2) im Wesentlichen abzudecken.

## Revendications

1. Dispositif de vaporisation personnel (1) comprenant :
une cartouche (2) qui inclut un réservoir (8) pour stocker un liquide à vaporiser, et
une partie corps allongée (4) à laquelle est raccordée la cartouche (2), dans lequel la partie corps allongée (4) comprend un élément de couverture (6) qui est mobile dans une direction longitudinale de la partie corps allongée (4) entre une première position (A) et une seconde position (B),
dans lequel la cartouche (2) est au moins partiellement recouverte ou dissimulée par l'élément de couverture (6) dans la première position (A),
**caractérisé en ce que** la cartouche (2) comprend un logement (7) qui renferme le réservoir (8),
la cartouche (2) est configurée pour être raccordée à une région d'extrémité (3) de la partie corps allongée (4) et dans lequel un embout (9) est fourni sur la cartouche (2).

2. Dispositif de vaporisation personnel (1) selon la revendication 1, dans lequel un volume de liquide dans le réservoir (8) de la cartouche (2) reste visible quand l'élément de couverture (6) est dans la première position (A).

3. Dispositif de vaporisation personnel (1) selon la revendication 2, dans lequel une fenêtre est fournie dans l'élément de couverture (6) et/ou une région latérale (S) de la cartouche (2) reste non recouverte par l'élément de couverture (6) pour qu'un utilisateur détermine visuellement la quantité de liquide qu'il reste dans le réservoir (8) de la cartouche (2) quand l'élément de couverture (6) est dans la première position (A).

4. Dispositif de vaporisation personnel (1) selon une quelconque revendication précédente, dans lequel la cartouche (2) inclut une fenêtre (W) pour qu'un utilisateur détermine visuellement la quantité de liquide qu'il reste dans le réservoir (8) de la cartouche (2), la fenêtre (W) étant fournie de préférence sur une région latérale (S) de la cartouche (2).

5. Dispositif de vaporisation personnel (1) selon une quelconque revendication précédente, dans lequel la seconde position (B) de l'élément de couverture (6) est une position sensiblement rétractée dans laquelle la cartouche (2) est sensiblement non recouverte ou non dissimulée par l'élément de couverture (6), pour la connexion de la cartouche (2) à, et/ou le retrait de la cartouche (2) de, la partie corps allongée (4), la seconde position (B) étant de préférence une position dans laquelle le dispositif de vaporisation personnel (1) est activé.

6. Dispositif de vaporisation personnel (1) selon une quelconque revendication précédente, dans lequel la première position (A) de l'élément de couverture (6) est une position sensiblement étendue, la première position (A) étant de préférence une position dans laquelle le dispositif de vaporisation personnel (1) est désactivé.

7. Dispositif de vaporisation personnel (1) selon une quelconque revendication précédente, dans lequel l'élément de couverture (6) forme une partie d'un boîtier externe (5) de la partie corps allongée (4).

8. Dispositif de vaporisation personnel (1) selon la revendication 7, dans lequel l'élément de couverture (6) comprend au moins l'un parmi un panneau de couverture avant (6') qui s'étend sur un avant de la partie corps allongée (4) et un panneau de couverture arrière (6") qui s'étend sur un arrière de la partie corps allongée (4).

9. Dispositif de vaporisation personnel (1) selon une quelconque revendication précédente, comprenant en outre au moins un capteur pour déterminer une ou plusieurs conditions de fonctionnement du dispositif de vaporisation personnel (1), dans lequel le au moins un capteur est incorporé dans la cartouche (2) et/ou la partie corps allongée (4), et dans lequel le au moins un capteur comprend un capteur de température, un capteur de volume de liquide, un capteur de bouffée, ou un capteur gyroscopique.

10. Dispositif de vaporisation personnel (1) selon une quelconque revendication précédente, dans lequel l'élément de couverture (6) recouvre ou dissimule au moins 50 % d'une surface externe de la cartouche (2) dans la première position (A), de préférence au moins 80 % d'une surface externe de la cartouche (2), plus préférentiellement dans la plage de 80 % à 100 % de la cartouche (2).

11. Dispositif de vaporisation personnel (1) selon une quelconque revendication précédente, dans lequel la partie corps allongée (4) comprend un commutateur (17) connecté de manière fonctionnelle à l'élément de couverture (6) et configuré pour activer au moins une fonctionnalité quand l'élément de couverture (6) est déplacé jusqu'à la première position (A) et/ou jusqu'à la seconde position (B), et dans lequel la partie corps allongée (4) comprend au moins un indicateur (19), de préférence un indicateur éclairé, tel qu'une DEL, pour indiquer un état de fonctionnement du dispositif de vaporisation personnel (1).

12. Dispositif de vaporisation personnel (1) selon la revendication 11, dans lequel le commutateur (17) est configuré pour activer le dispositif de vaporisation personnel (1) pour générer une vapeur après que l'élément de couverture (6) a été déplacé jusqu'à la seconde position (B), et dans lequel le au moins un indicateur (19) est configuré pour indiquer que le dispositif de vaporisation personnel (1) a été activé.

13. Dispositif de vaporisation personnel (1) selon une quelconque revendication précédente, comprenant en outre un moyen de guidage (25) pour guider un déplacement de l'élément de couverture (6) dans la direction longitudinale entre la première position (A) et la seconde position (B).

14. Procédé d'installation d'une cartouche (2) amovible dans un dispositif de vaporisation personnel (1), en particulier dans un article électronique à fumer, comprenant les étapes consistant à :
fournir un dispositif de vaporisation personnel (1) présentant une partie corps allongée (4) à laquelle la cartouche (2) amovible est configurée pour être raccordée, la cartouche (2) amovible incluant un logement qui renferme un réservoir (8) pour stocker un liquide à vaporiser et un embout (9) fourni sur la cartouche (2) amovible ;
fournir un élément de couverture (6) sur la partie corps allongée (4) qui est mobile dans une direction longitudinale de la partie corps (4) entre une première position (A) étendue et une seconde position (B) rétractée ;
déplacer l'élément de couverture (6) vers la seconde position (B) rétractée pour accéder à une région d'extrémité (3) de la partie corps allongée (4) ; et
fixer la cartouche (2) amovible à la région d'extrémité (3) accédée de la partie corps allongée (4) du dispositif de vaporisation personnel (1).

15. Procédé selon la revendication 14, comprenant l'étape consistant à : après avoir fixé la cartouche (2) amovible à la région d'extrémité (3) de la partie corps allongée (4), déplacer l'élément de couverture (6) vers la première position (A) étendue pour recouvrir sensiblement la cartouche (2) amovible.
